# EUROPEAN PATENT APPLICATION

(11) **EP 4 458 335 A1**
(43) Date of publication of application: **06.11.2024**
(21) Application number: 24173997.8
(22) Date of filing: 03.05.2024
(51) Int. Cl.: A61F 13/15

(54) **METHOD AND DEVICE FOR PROCESSING DISCRETE SEGMENTS FOR ABSORBENT ARTICLES**

(30) Priority: 05.05.2023 IT 202300008976
(71) Applicant: GDM S.P.A., 40133 Bologna (IT)
(72) Inventor: SACCOMANI, Alessandro, Bologna (IT); ZAVALLONI, Alessandro, Bologna (IT)
(74) Representative: Casadei, Barbara

(57) **Abstract**

A method for processing a discrete segment (10) of material for absorbent articles comprises a step of picking up the segment (10) from a first succession (S1) of segments (10) disposed at a first spacing (P1) from each other;
a step of releasing the segment (10) to define a second succession (S2) of segments (10) disposed at a second spacing (P2) from each other;
a step of rotating the segment (10) carried out between the step of picking up and the step of releasing and which involves disposing the segment (10) from a first configuration, corresponding to the configuration of the segment (10) of the first succession (S1), to a second configuration, rotated relative to the first configuration and corresponding to the configuration of the segment (10) of the second succession (S2);
between the step of picking up and the step of releasing there is a step of accelerating the segment (10) and a step of decelerating the segment (10); the step of rotating the segment (10) is carried out simultaneously with at least one between the step of accelerating and the step of decelerating.

## Description

This invention relates to a method and a device for processing discrete segments for absorbent articles.

The term "discrete segment for absorbent articles" is used, for exemplary, non-limiting purposes, to denote an absorbent pad or absorbent product (with any structure) having one or more superposed layers of nonwoven/Loft/High Loft or other fabric.

The method and device for processing discrete segments for absorbent articles disclosed in this specification refer to variators for changing the orientation of the segments by rotating them from a configuration in which the axis of longitudinal extension of each segment is parallel to the feed direction to a configuration in which the axis of longitudinal extension is transverse to the feed direction, in particular, rotated by 90°.

Since the segments have to be positioned with their axes of longitudinal extension perpendicular to the feed direction, there is a need to reduce the spacing between one segment and another.

Taking into consideration the size of a segment and, in particular, its longitudinal dimension, the longer the segment is, the more the spacing must be reduced.

In prior art solutions in which the orientation of discrete segments needs to changed while at the same time changing the spacing of the succession of segments from the spacing before being rotated to that after being rotated, the segments are rotated by a rotary means and the segment spacing is varied by a series of accelerators located downstream of the rotary means. In a market where machines are focused more and more on greater flexibility and compactness in offering product size changeover capabilities, the need was felt to provide a method and a device capable of processing the segments to change their orientation and simultaneously changing their spacing.

The need was also felt to provide a method which allows processing different sizes by replacing just some of the components of the device, without having to replace the entire device as in the prior art.

Thus, the procedure for changeover from one size to another is much quicker and fewer components are involved in machine installation.

The method for processing a discrete segment of material for absorbent articles comprises a step of picking up the segment from a first succession of segments disposed at a first spacing from each other.

A step of releasing the segment to define a second succession of segments disposed at a second spacing from each other.

A step of rotating the segment carried out between the step of picking up and the step of releasing and which involves disposing the segment from a first configuration, corresponding to the configuration of the segment of the first succession, to a second configuration, rotated relative to the first configuration and corresponding to the configuration of the segment of the second succession.

The method comprises, between the step of picking up and the step of releasing, a step of accelerating the segment and a step of decelerating the segment.

The step of rotating the segment is carried out simultaneously with at least one between the step of accelerating and the step of decelerating.

Preferably, according to the method, the step of rotating the segment is carried out at the same time as the step of accelerating and the step of decelerating.

Preferably, according to the method, the step of changing the size of the segment involves varying the duration of the step of accelerating and the magnitude of the acceleration and varying the duration of the step of decelerating and the magnitude of the deceleration.

Preferably, the first spacing is greater than the second spacing.

Alternatively, the first spacing is less than the second spacing.

Alternatively, the first spacing is equal to the second spacing.

Preferably, the step of rotating the segment comprises rotating it by at least 90°.

Preferably, the step of accelerating the segment is carried out before the step of decelerating the segment or after it.

Preferably, the method is implemented by a single device for processing a segment having a working circumference corresponding to the size of a segment to be processed.

Also an object of this invention is a device for processing a discrete segment for absorbent articles, comprising a rotary means, which rotates about its own axis of rotation and which is configured to move a plurality of pads at least from a pickup station, for picking up a respective segment, to a releasing station for releasing the segment, where the segments at the pickup station are fed at a first spacing and the segments at the releasing station are released at a second spacing.

The rotary means is configured to move the pads along a working circumference corresponding to the size of a segment to be processed.

First motion transmission means are configured to control a relative rotation of each pad about its own axis of rotation so that each pad passes from a first configuration to a second configuration which is rotated relative to the first configuration, and vice versa.

Second motion transmission means are configured to control an oscillation of each pad about a respective axis of oscillation.

The second motion transmission means are configured to control an oscillation that is consistent with the rotation direction of the rotary means and an oscillation that is inconsistent with the rotation direction of the rotary means.

Preferably, the oscillation of the pad consistent with the rotation direction of the rotary means has a range of angular movement of ±90°.

Preferably, the oscillation of the pad inconsistent with the rotation direction of the rotary means has a range of angular movement of ±90°.

Preferably, each pad has a longitudinal dimension included in a range from 100 mm to 600 mm and a transverse dimension included in a range from 30 mm to 300 mm.

Preferably, the first transmission means comprise a plurality of motion transmission members, each associated with a respective pad.

Each motion transmission member comprises a first arm and a second arm, each free to rotate about its own axis of rotation, parallel to the axis of oscillation of the respective pad.

The first arm is kinematically connected to the pad and the second arm is kinematically connected to the first transmission means.

The first arm and the second arm are connected to each other through rolling means, configured to allow the first arm and the second arm to rotate freely about the respective axes of rotation, and transmission means for transmitting an axial force whose direction is parallel to the direction of the axes of rotation of the first arm and second arm.

Preferably, the first motion transmission means are configured to control a relative rotation of each pad about its own axis of rotation so that each pad passes from a first configuration to a second configuration which is rotated by at least 90°.

Further features and advantages of this invention are more apparent in the following detailed description of preferred embodiments of it, with reference to the accompanying drawings, in which:

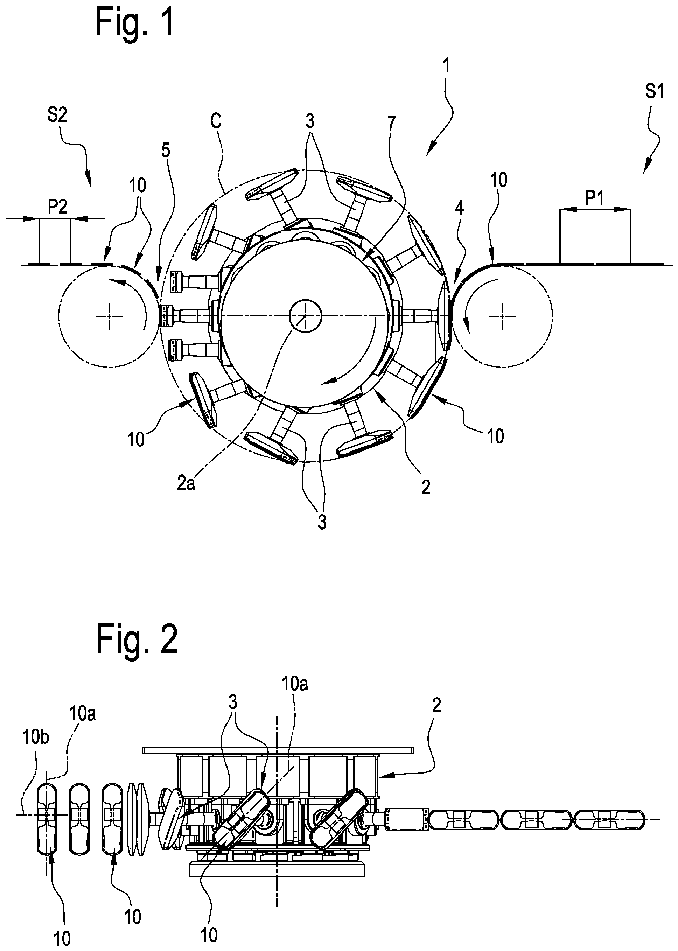

- Figure 1 is a schematic front view of a device for processing discrete segments according to this invention;
- Figure 2 shows a schematic perspective view of a detail from Figure 1;

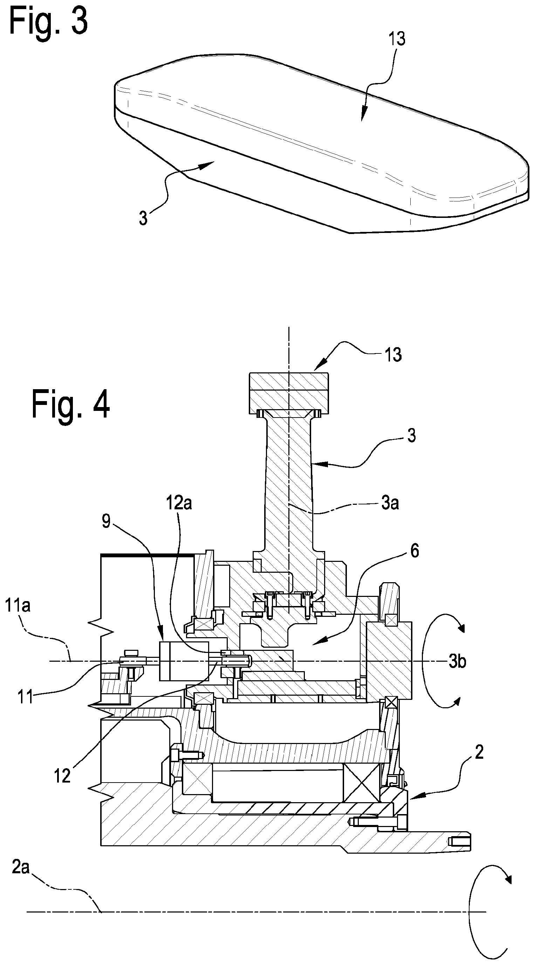

- Figure 3 shows a detail of the device of Figure 1 in a schematic cross sectional view, with some parts cut away in order to better illustrate others;
- Figure 4 shows a schematic perspective view of a detail from Figure 3;

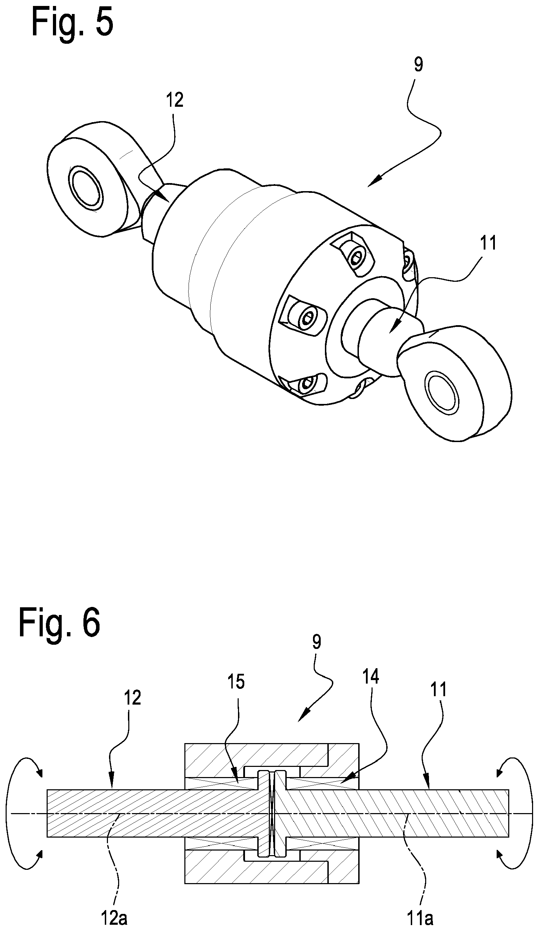

- Figure 5 shows a schematic cross section of the detail of Figure 4.

The numeral 1 denotes a device for processing a discrete segment 10 for absorbent articles.

The device 1 comprises a rotary means 2 configured for moving a plurality of pads 3 at least from a pickup station 4 for picking up a respective segment 10 to a releasing station 5 for releasing the segment 10.

The pickup station 4 and the releasing station 5 are located along the same working circumference C.

That means the pickup station 4 and the releasing station 5 are both the same distance from the axis of rotation 2a of the rotary means 2.

The rotary means 2 rotates about its own axis of rotation 2a, entraining the pads 3 in rotation about said axis.

The rotary means 2 is configured to move the pads 3 along a working circumference C corresponding to the size of a segment 10 to be processed.

The axis of rotation 2a is a horizontal axis.

At the pickup station 4, the segments 10 are fed at a first spacing P1.

At the releasing station 5, in the embodiment illustrated, the segments 10 are released at a second spacing P2, smaller than the spacing P1.

In alternative embodiments not illustrated, the first spacing P1 is smaller than the second spacing P2, or alternatively, the first spacing P1 is equal to the second spacing P2.

Each segment 10 is in the form of a planar element having a longitudinal dimension and a transverse dimension.

The longitudinal dimension is variable in a range from 100 to 600 cm.

The transverse dimension is variable in a range from 30 to 300 cm.

A longitudinal axis 10a and a transverse axis 10b are identifiable in each segment 10.

The segment 10 of this disclosure is rectangular in shape and extends predominantly along the longitudinal axis 10a.

This disclosure does not exclude that the segment 10 may have other shapes, for example, a square shape.

With reference to the pickup station 4, the segments 10 are disposed with their respective longitudinal axes 10a parallel to the feed direction, defining a first succession with spacing P1.

With reference to the releasing station 5, the segments 10 are disposed with their respective longitudinal axes 10a transverse, in particular perpendicular, to the feed direction, defining a second succession with spacing P2.

That is because the device 1 comprises first motion transmission means 6 which are configured to control a relative rotation of each pad 3 about its own axis of rotation 3a so as to turn the pad 3 from a first configuration to a second configuration, rotated by 90° relative to the first configuration, and vice versa.

In order to reduce the spacing from the first spacing P1 to the second spacing P2, second motion transmission means 7 are configured to control an oscillation of each pad 3 about a respective axis of oscillation 3b.

It should be noted that in a preferred embodiment, the axis of oscillation 3b is parallel to the axis of rotation 2a of the rotary means 2.

The axis of rotation 3a of each pad 3 is transverse to, and intersects the axis of oscillation 3b of the pad 3.

The second motion transmission means 7 are configured to sequentially control an oscillation of each pad 3 which is consistent with the rotation direction of the rotary means 2, where this oscillation has a range of angular movement of 90°, and an oscillation of each pad 3 that is inconsistent with the rotation direction of the rotary means 2, where this oscillation has a range of angular movement of 90°.

It should be noted that during the oscillation of each pad 3, in a direction consistent with and in a direction inconsistent with the rotation direction of the rotary means 2, the pad 3 varies its position relative to the axis of rotation 2a of the rotary means 2.

In other words, each pad 3, during its rotation about the axis of rotation 2a of the rotary means 2, does not remain at a fixed position on the working circumference C, except when positioned at the pickup station 4 and at the releasing station 5 where the pad 3 is disposed at a substantially radial position relative to the axis of rotation 2a of the rotary means 2.

Advantageously, the oscillation of each pad 3 consistently with the rotation direction of the rotary means 2 allows imparting an acceleration to the pad 3 which has picked up a segment 10, this acceleration making it possible to prevent the pads 3 from colliding as they rotate about their axes of rotation 3a and to reduce the spacing between the rotated segments 10 which are released.

The oscillation in the inconsistent direction is necessary to slow down the pad 3 to return it to the working circumference C where the releasing station 5 is located.

Another advantage is that a plurality of sizes of segments 10 can be processed with a single device 1 since the working circumference C is set for one segment 10 and the other sizes of segment 10 are controlled accordingly by varying the amplitude of the oscillations of the pads 3.

In other words, for each size of segment 10 there is a corresponding amplitude range of the oscillation consistent and inconsistent with the rotation direction of the rotary means 2 and a corresponding amplitude range.

Preferably, the second transmission means 7 comprise cam means 8 configured to control the law of motion of the pads 3.

The cam means 8 are configured to impart the oscillation of each pad 3 in the direction consistent with the rotation direction of the rotary means 2 and in the direction inconsistent with the rotation direction of the rotary means 2, according to the size of the segment 10.

Advantageously, the device 1 according to this invention allows a plurality of sizes of segment 10 to be processed because the working circumference C always passes through the pickup station 4 and the releasing station 5.

Each pad 3 has a longitudinal dimension included in a range from 100 mm to 600 mm and a transverse dimension included in a range from 30 mm to 300 mm.

Preferably, each pad 3 has a curvature radius along a longitudinal direction and a curvature radius along a transverse direction.

Preferably, each pad 3 has a trapezoidal profile defined by a central portion which is rectangular in shape and two end portions which are contiguous with the central portion on opposite sides of it and which are inclined relative to the central portion.

Advantageously, this makes it easier for the segment 10 to be picked up and released in both configurations of the pad 3, namely, the first, pickup configuration and the second, releasing configuration which is rotated by 90° relative to the first configuration, since the pad 3, relative to the axis of rotation 2a of the rotary means 2, is located at a radial position when it is at the pickup station 4 and at the releasing station 5.

The pads 3 have a support surface 13 to which the segment 10 is held by suction from the pickup station 4 to the releasing station 5.

Preferably, to make it easier for the segment 10 to be expelled from the support surface 13 of the pad 3, blowing means, not illustrated, are provided.

As stated above, each pad 3 of the device 1 performs a rotation about the axis of rotation 2a of the rotary means 2, a rotation about its own axis of rotation 3a to pass from the first configuration to the second configuration (and vice versa), an oscillation about is own axis 3b in a direction consistent with the rotation direction of the rotary means 2 and an oscillation about is own axis 3b in a direction inconsistent with the rotation direction of the rotary means 2.

It should be noted that, in use, some of these movements are carried out simultaneously; starting from the pickup station 4, the pad 3 which has picked up a respective segment 10 is carried in rotation by the rotary means 2 about the axis of rotation 2 of the rotary means 2, towards the releasing station 5 so it can be released.

During the rotation about the axis of rotation 2a of the rotary means 2, each pad 3 is carried in rotation about the respective axis of rotation 3a to pass from the first configuration to the second configuration, rotated by 90° relative to the first configuration.

During the rotation about its own axis of rotation 3, each pad 3 is commanded to perform the oscillation in the direction consistent with the rotation direction of the rotary means 2, and the oscillation in the direction inconsistent with the rotation direction of the rotary means 2.

From the releasing station 5 to the pickup station 4, during the rotation about the axis of rotation 2a of the rotary means 2, each pad 3 is carried in rotation about the respective axis of rotation 3a to pass from the second configuration to the first configuration to get ready to pick up another segment 10.

In order to be able to control the rotation of the pad 3 about its own axis of rotation 3a simultaneously with the oscillation of the pad 3, both consistently and inconsistently with the rotation direction of the rotary means 2, the first transmission means 6 comprise a plurality of motion transmission members 9, each associated with a respective pad 3.

Each motion transmission member 9 comprises a first arm 11 and a second arm 12, each free to rotate about its own axis of rotation 11a, 12a, parallel to the axis of oscillation 3b of the respective pad 3.

The first arm 11 is kinematically connected to the pad 3 and the second arm 12 is kinematically connected to the first transmission means 6.

The first arm 11 and the second arm 12 are connected to each other through rolling means 15, configured to allow the first arm 11 and the second arm 12 to rotate freely about the respective axes of rotation 11a, 12a, and transmission means 14 for transmitting an axial force whose direction is parallel to the direction of the axes of rotation 11a, 12a of the first arm 11 and second arm 12.

The transmission means 14 for transmitting the axial force are configured to allow the second arm 12 to impart a force in first direction to drive the pad 3 to pass from the first configuration to the second configuration, and in a second direction, opposite of the first direction, to drive the pad 3 to pass from the second configuration to the first configuration.

Preferably, the force in the first direction is a pushing force and the force in the second direction is a pulling force.

Advantageously, since the first arm 11 and the second arm 12 are free to rotate about the respective axes 11a, 12a, they may be disposed at different angular positions when the pushing force and the pulling force are being applied, because the first arm 11 is following the oscillation of the pad 3 to which it is kinematically connected, while the second arm 12 is relatively stationary relative to it.

This invention also relates to a method for processing a discrete segment 10 of material for absorbent articles, comprising a step of picking up the segment 10 from a first succession S1 of segments 10 disposed at a first spacing (P1) from each other.

A step of releasing the segment 10 to define a second succession S2 of segments 10 disposed at a second spacing P2 from each other.

The first spacing P1 is greater than the second spacing P2 or that the first spacing P1 is smaller than the second spacing P2 or that the first spacing P1 is equal to the second spacing P2.

From the step of picking up to the step of releasing, the segment 10 is carried in rotation at a variable rotation speed.

A step of rotating the segment 10 carried out between the step of picking up 4 and the step of releasing and involves disposing the segment 10 from a first configuration, corresponding to the configuration of the segment 10 of the first succession S1, to a second configuration, rotated relative to the first configuration and corresponding to the configuration of the segment 10 of the second succession S2.

The step of rotating the segment 10 comprises rotating it by at least 90°.

In sequence from the step of picking up to the step of releasing, the method comprises a step of accelerating the segment 10 and a step of decelerating the segment 10.

The step of accelerating the segment 10 is carried out before or after the step of decelerating the segment 10.

The step of rotating the segment 10 is carried out simultaneously with at least one between the steps of accelerating and decelerating the segment 10.

In the embodiment of this invention, the step of rotating the segment 10 is carried out simultaneously with the step of accelerating and with the step of decelerating.

The method comprises a step of changing the size of the segment 10, which involves varying the duration of the step of accelerating and varying the duration of the step of decelerating.

The method is implemented by a single device for processing a segment 10 having a working circumference C corresponding to the size of a segment 10 to be processed.

## Claims

1. A method for processing a discrete segment (10) of material for absorbent articles, comprising
a step of picking up the segment (10) from a first succession (S1) of segments (10) disposed at a first spacing (P1) from each other;
a step of releasing the segment (10) to define a second succession (S2) of segments (10) disposed at a second spacing (P2) from each other;
a step of rotating the segment (10) carried out between the step of picking up and the step of releasing and which involves disposing the segment (10) from a first configuration, corresponding to the configuration of the segment (10) of the first succession (S1), to a second configuration, rotated relative to the first configuration and corresponding to the configuration of the segment (10) of the second succession (S2);
a step of implementing the method by means of a single device for processing a segment (10) having a working circumference (C) corresponding to the size of a segment (10) to be processed;
the method is **characterized in that** it comprises, between the step of picking up and the step of releasing, a step of accelerating the segment (10) and a step of decelerating the segment (10); the step of rotating the segment (10) is carried out simultaneously with at least one between the step of accelerating and the step of decelerating.

2. The method according to the preceding claim, **characterized in that** the step of rotating the segment (10) is carried out simultaneously with the step of accelerating and the step of decelerating.

3. The method according to either of the preceding claims, **characterized in that** it comprises a step of changing the size of the segment (10), which involves varying the duration of the step of accelerating and the magnitude of the acceleration and varying the duration of the step of decelerating and the magnitude of the deceleration.

4. The method according to any one of the preceding claims, **characterized in that** the first spacing (P1) is greater than the second spacing (P2) or that the first spacing (P1) is smaller than the second spacing (P2) or that the first spacing (P1) is equal to the second spacing (P2).

5. The method according to any one of the preceding claims, **characterized in that** the step of rotating the segment (10) comprises rotating it by at least 90°.

6. The method according to any one of the preceding claims, **characterized in that** the step of accelerating the segment (10) is carried out before or after the step of decelerating the segment (10).

7. A device for processing a discrete segment (10) of material for absorbent articles, comprising a rotary means (2), rotating about its own axis of rotation (2a) and configured to move a plurality of pads (3) at least from a pickup station (4) for picking up a respective segment (10) to a releasing station (5) for releasing the segment (10),
the rotary means (2) is configured to move the pads (3) along a working circumference (C) corresponding to the size of a segment (10) to be processed;
at the pickup station (4), the segments (10) are fed at a first spacing (P1); at the releasing station (5), the segments (10) are released at a second spacing (P2);
first motion transmission means (6) are configured to control a relative rotation of each pad (3) about its own axis of rotation (3a) so that each pad (3) passes from a first configuration to a second configuration which is rotated relative to the first configuration, and vice versa;
the device being **characterised in that** it comprises
second motion transmission means (7) configured to control an oscillation of each pad (3) about a respective axis of oscillation (3b);
the second motion transmission means (7) are configured to control an oscillation that is consistent with the rotation direction (2a) of the rotary means (2) and an oscillation that is inconsistent with the rotation direction (2a) of the rotary means (2).

8. The device according to the preceding claim, **characterized in that** the oscillation of the pad (3) consistent with the rotation direction (2a) of the rotary means (2) has a range of angular movement of ±90°.

9. The device according to claim 7 or 8, **characterized in that** the oscillation of the pad (3) inconsistent with the rotation direction (2a) of the rotary means (2) has a range of angular movement of ±90°.

10. The device according to any one of claims 7 to 9, **characterized in that** each pad (3) has a longitudinal extent included in a range between 100 mm and 600 mm and a transverse extent included in a range between 30 mm and 300 mm.

11. The device according to any one of claims 7 to 10, **characterized in that** the first transmission means (6) comprise a plurality of motion transmission members (9), each associated with a respective pad (3); each motion transmission member (9) comprises a first arm (11) and a second arm (12), each free to rotate about its own axis of rotation (11a, 12a), parallel to the axis of oscillation (3b) of the respective pad (3); the first arm (11) is kinematically connected to the pad (3) and the second arm (12) is kinematically connected to the first transmission means (6); the first arm (11) and the second arm (12) are connected to each other through rolling means (15), configured to allow the first arm (11) and the second arm (12) to rotate freely about the respective axes of rotation (11a, 12a), and transmission means (14) for transmitting an axial force whose direction is parallel to the direction of the axes of rotation (11a, 12a) of the first arm (11) and second arm (12).

12. The device according to any one of claims 7 to 11, **characterized in that** first motion transmission means (6) are configured to control a relative rotation of each pad (3) about its own axis of rotation (3a) so that each pad (3) passes from a first configuration to a second configuration which is rotated by at least 90°.

13. The device according to any one of claims 7 to 12, **characterized in that** the first spacing (P1) is greater than the second spacing (P2) or that the first spacing (P1) is smaller than the second spacing (P2) or that the first spacing (P1) is equal to the second spacing (P2).
